Europäisches Patentamt

European Patent Office

Office européen des brevets

(11) Publication number: **0 080 827**
**B1**

(12) **EUROPEAN PATENT SPECIFICATION**

(45) Date of publication of patent specification: **09.03.88**

(21) Application number: **82306125.4**

(22) Date of filing: **17.11.82**

(51) Int. Cl.⁴: **C 12 P 13/00, A 01 N 37/38 //**
**C12N9/18**

(54) Method for biotechnologically preparing (S)-(-)-alpha-cyano-3-phenoxybenzyl alcohol.

(30) Priority: **28.11.81 JP 191340/81**
**02.06.82 JP 95207/82**

(43) Date of publication of application:
**08.06.83 Bulletin 83/23**

(45) Publication of the grant of the patent:
**09.03.88 Bulletin 88/10**

(84) Designated Contracting States:
**CH DE FR GB IT LI NL**

(56) References cited:
**EP-A-0 036 774**
**FR-A-2 053 218**
**FR-A-2 295 933**
**FR-A-2 447 899**
**US-A-3 607 651**

**AGRICULTURAL & BIOLOGICAL CHEMISTRY,**
**vol. 45, no. 6, June 1981, pages 1389-1392,**
**Tokyo, JP SHINOBU IRIUCHIJIMA et al.:**
**"Asymmetric hydrolysis of (+/-)-alpha-**
**substituted carboxylic acid esters with**
**microorganisms"**

(73) Proprietor: **SUMITOMO CHEMICAL COMPANY,**
**LIMITED**
**15 Kitahama 5-chome Higashi-ku**
**Osaka-shi Osaka 541 (JP)**

(72) Inventor: **Mitsuda, Satoshi**
**1-26-409, Tamagawa**
**Takatsuki-shi Osaka 569 (JP)**
Inventor: **Hirohara, Hideo**
**31-18, Takakuradai 4-cho**
**Sakai-shi Osaka 590-01 (JP)**

(74) Representative: **Myerscough, Philip Boyd et al**
**J.A.Kemp & Co. 14, South Square Gray's Inn**
**London, WC1R 5EU (GB)**

(56) References cited:

**PATENTS ABSTRACTS OF JAPAN, vol. 5, no.**
**111 (C-63)783r, 18th July 1981**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a method for biotechnologically preparing (S)-(−)-α-cyano-3-phenoxybenzyl alcohol. More particularly, it relates to a method for preparing (S)-(−)-α-cyano-3-phenoxybenzyl alcohol according to a biotechnological process, wherein an organic carboxylic acid (saturated or unsaturated, having 1 to 18 carbon atoms) ester of (R,S)-α-cyano-3-phenoxybenzyl alcohol is reacted with an esterase originated from a microorganism or an animal pancreas or liver, which is capable of asymmetrically hydrolyzing the ester of the (S)-(−)-α-cyano-3-phenoxybenzyl alcohol predominantly, at not higher than pH 7 to asymmetrically hydrolyze the ester to give the (S)-(−)-α-cyano-3-phenoxybenzyl alcohol and the ester of its antipode.

α-Cyano-3-phenoxybenzyl alcohol has been known as a novel alcohol moiety of a series of ester compounds of synthetic pyrethroids, having superior insecticidal activity. The synthetic pyrethroids, typically, fenvalerate, cypermethrin, deltamethrin, etc., which have the said alcohol as their alcohol moiety, are good insecticides not only for household and sanitary uses but even for agricultural use, since they provide the durability against the sun-light together with their leaping increase in the effectiveness. Thus, the importance of this α-cyano-3-phenoxybenzyl alcohol is more increasing.

Now, α-cyano-3-phenoxybenzyl alcohol has an asymmetric carbon at its α-position, accordingly having two kinds of optical isomers, i.e. (S)- and (R)-isomers. When they are derived to the esters, it has been known that the insecticidal activity of the ester from the (S)-isomer alcohol is far superior to that from the (R)-isomer alcohol. (See Hirosuke Yoshioka; Journal of Synthetic Organic Chemistry, Japan, Vol. 38, No. 12, pages 1151—1162, 1980). Therefore, the development of a commercially advantageous technology is desirable in an optical resolution of (R,S)-α-cyano-3-phenoxybenzyl alcohol, which is prepared according to the conventional method of synthetic chemistry, in order to obtain the (S)-isomer.

While, α-cyano-3-phenoxybenzyl alcohol is an unstable compound. Thus, the conventional optical resolution process for alcohol which is effected via an ester or a half-ester with an ordinary optically active organic acid may not be advantageous for it. As the methods to obtain (S)-(−)-α-cyano-3-phenoxybenzyl alcohol, the following methods have been known; (1) a method wherein desired (S)-(−)-α-cyano-3-phenoxybenzyl alcohol is obtained by reacting (R,S)-α-cyano-3-phenoxybenzyl alcohol with cis-2,2-dimethyl-3(S)-(dihydroxymethyl)-cyclopropane-1(R)-carboxylic acid lactone in the presence of an acidic reagent to obtain an ether compound, separating the resulting mixture of two kinds of the isomers each other according to a physical means, and hydrolysing the ether compound containing (S)-(−)-isomer alcohol component in an acidic medium (see Japanese Unexamined Published Patent Application No. 109944/1979); and (2) a method wherein (S)-(−)-α-cyano-3-phenoxybenzyl alcohol is obtained by reacting an (S)-(−)-α-cyano-3-phenoxybenzyl alcohol ester of a chiral cyclopropanecarboxylic acid with a boron halide and then with water (see Japanese Unexamined Published Patent Application No. 12355/1981).

In these methods, however, the processes are complicated and need an expensive optically active reagent, besides, with not so high overall yield. Furthermore, in the method (2), the optically active α-cyano-3-phenoxybenzyl alcohol ester has to be prepared in advance. From these points, the methods heretofore known can not necessarily be said satisfactory ones.

After the studies to overcome these difficulties and to seek a commercially advantageous method for preparing (S)-(−)-α-cyano-3-phenoxybenzyl alcohol, the present inventors have found the fact that (S)-(−)-α-cyano-3-phenoxybenzyl alcohol can be produced efficiently from an ester of (R,S)-α-cyano-3-phenoxybenzyl alcohol, as the starting material, and asymmetrically hydrolyzing it according to a biotechnological process. Thus, we have accomplished the present invention based upon such findings, with the other results of various further investigations.

Accordingly, the present invention provides a method for preparing (S)-(−)-α-cyano-3-phenoxybenzyl alcohol, wherein an organic carboxylic acid (saturated or unsaturated, having 1 to 18 carbon atoms) ester of (R,S)-α-cyano-3-phenoxybenzyl alcohol is subjected to asymmetric hydrolysis using an esterase originated from a microorganism or an animal pancreas or liver, which is capable of asymmetrically hydrolyzing the ester of the (S)-(−)-α-cyano-3-phenoxybenzyl alcohol predominantly, at not higher than pH 7 to asymmetrically hydrolyze the ester, thereby to give the (S)-(−)-α-cyano-3-phenoxybenzyl alcohol and the ester of its antipode.

As the organic carboxylic acid moieties composing the esters with (R,S)-α-cyano-3-phenoxybenzyl alcohol which are employed as the starting material in the present invention, there may be illustrated, for example, formic, acetic, propionic, butyric, valeric, isovaleric, caproic, caprylic, capric, lauric, myristic, palmitic, stearic, oleic, linolic acids, etc. From the viewpoints of easiness in handling and of the optical purity of the reaction product, esters of organic carboxylic acids having 2 to 12 carbon atoms are preferred as the starting material. From the viewpoints of economy, furthermore, along with the easiness in handling, the ester of acetic acid is the most preferred.

The starting ester may easily be prepared according to the conventional esterification process, for example, by reacting (R,S)-α-cyano-3-phenoxybenzyl alcohol with an anhydride or a halide of such organic carboxylic acid. The ester may also be prepared by reacting 3-phenoxybenzaldehyde and sodium cyanide, with an anhydride or a halide of such organic carboxylic acid.

The term "esterase" used herein is of their broader meaning including lipase having activity to a water-insoluble substrate. The esterases employable in the present invention are those capable of

2

**0 080 827**

asymmetrically hydrolyzing the ester of the (S)-(−)-α-cyano-3-phenoxybenzyl alcohol predominantly and they are originated from microorganisms and from animal pancreases or livers.

With respect to the microorganisms producing the esterases employable in the present invention, any microorganisms may be used as far as they produce the esterases capable of asymmetrically hydrolyzing the ester of the (S)-(−)-α-cyano-3-phenoxybenzyl alcohol predominantly.

The example of genuses of such microorganisms are as follows:

*Arthrobacter, Alcaligenes, Achromobacter, Pseudomonas, Aspergillus, Mucor, Chromobacterium, Bacillus, Micrococcus, Candida, Torulopsis, Nocardia, Rhodotorula, Brevibacterium, Enterobacter, Flavobacterium, Mycobacterium, Corynebacterium, Lactobacillus, Streptomyces, Trichoderma, Penicillium, Rhizopus, Aureobasidium, Actinomucor, Gibberella, Geotricum, Absidia, Cunninghamella, Gliocladium, Saccharomyces, Cryptococcus, Pichia,* or *Hansenula.*

In these microorganisms, furthermore, the esterase originated from the microorganisms belonging to the following genuses are preferred from the viewpoints of the optical selectivity and the ability of hydrolysis.

*Arthrobacter, Alcaligenes, Achromobacter, Pseudomonas, Aspergillus, Chromobacterium, Candida, Torulopsis, Nocardia, Rhodotorula, Brevibacterium, Mycobacterium, Streptomyces, Trichoderma, Rhizopus* or *Geotrium.*

Such microorganisms may be cultured in a liquid medium according to the conventional procedure. For example, a microorganism may be inoculated to a sterilized liquid medium (e.g., a bouillon medium for bacteria, a malt extract—yeast extract medium for fungi and yeasts), and subjected to a shaken culture ordinarily at a temperature of 20° to 40°C for 2 to 3 days. If necessary, it may be cultured on a solid medium.

Some of the microorganism-originated esterases have been commercially available, which can also be employed to fulfill the object of the present invention. The commercially available enzymes employable in the invention are illustrated below.

| Lipase | Origin | Seller or manufacturer |
|---|---|---|
| Lipase AP | *Aspergillus* sp. | Amano Seiyaku |
| Lipase M-AP | *Mucor* sp. | " |
| Lipase "Amano" | *Pseudomonas* sp. | " |
| Lipase Godo BSL | *Arthrobacter ureafaciens* nov. *var.* | Godo Shusei |
| Lipase Toyo | *Chromobacterium viscosum var. paralipolyticum* | Toyo Jozo |
| Lipase AL | *Achromobacter* sp. | Meito Sangyo |
| Lipase PL No. 266 | *Alcaligenes* sp. | " |
| Lipase PL No. 679 | *Alcaligenes* sp. | " |
| Steapsin | Hog pancreas | Tokyo Kasei |
| Pancreatin | Hog pancreas | " |
| Esterase | Hog liver | Sigma |

Other examples of microorganisms employed in the present invention are the strains as mentioned below, which have been deposited in American Type Culture Collection, U.S.A., (ATCC) or Institute for Fermentation, Osaka, Japan, (IFO) and are available from such depositories.

3

| | | | |
|---|---|---|---|
| (1) | Chromobacterium viscosum | ATCC | 6918 |
| (2) | Bacillus licheniformis | IFO | 12197 |
| (3) | Micrococcus luteus | IFO | 3066 |
| (4) | Candida utilis | IFO | 1086 |
| (5) | Torulopsis candida | IFO | 0380 |
| (6) | Nocardia erythropolis | IFO | 12320 |
| (7) | Rhodotorula minuta | IFO | 0387 |
| (8) | Brevibacterium ammoniagenes | IFO | 12072 |
| (9) | Enterobacter cloacae | IFO | 3320 |
| (10) | Flavobacterium arborescens | IFO | 3750 |
| (11) | Mycobacterium phlei | IFO | 3158 |
| (12) | Corynebacterium equi | ATCC | 7699 |
| (13) | Lactobacillus casei | IFO | 3322 |
| (14) | Streptomyces griseus | IFO | 3356 |
| (15) | Trichoderma viride | IFO | 4847 |
| (16) | Penicillium frequentans | IFO | 5692 |
| (17) | Rhizopus chinensis | IFO | 4737 |
| (18) | Aureobasidium pullulans | IFO | 4464 |
| (19) | Actinomucor elegans | IFO | 4022 |
| (20) | Gibberella zeae | IFO | 7160 |
| (21) | Geotricum candidum | IFO | 5368 |
| (22) | Absidia hyalospora | IFO | 8082 |
| (23) | Cunninghamella elegans | IFO | 6334 |
| (24) | Gliocladium roseum | IFO | 5422 |
| (25) | Saccharomyces ruoxii | IFO | 0505 |
| (26) | Cryptococcus albidus | IFO | 0378 |
| (27) | Pichia polimorpha | IFO | 1166 |
| (28) | Hansenula anomala | IFO | 0117 |

In practicing the method of the present invention, the asymmetric hydrolysis of the organic carboxylic acid ester of (R,S)-α-cyano-3-phenoxybenzyl alcohol is conducted by stirring or shaking vigorously a mixture of the said (R,S)-ester and an esterase-containing liquor, such as cultured liquor of such microorganism, its filtrate, esterase extracted liquor and its concentrate, suspension of microorganism cells or aqueous solution containing crude or purified esterase preparation or animal pancreas or liver esterase preparation. If required, surfactant of non-ester type may be added thereto. Alternatively, the enzyme may be employed in an immobilized state.

In this process, the reaction temperature is suitably in a range of 10° to 65°C. More preferable

4

temperature is in a range of 20° to 50°C, since the resulting alcohol is comparatively unstable at a higher temperature and the reaction rate is low at a lower temperature.

The period of time for the reaction is normally in a range of 3 to 48 hours, but it can be shortened at a higher reaction temperature or by use of an enzyme of high activity. It is essential to control the pH of the aqueous solution during the asymmetric hydrolysis reaction, because α-cyano-3-phenoxybenzyl alcohol tends to decompose especially by a basic substance. In a basic medium, the resulting alcohol is subjected to decomposition, though the asymmetric hydrolysis proceeds well by esterase. Accordingly, the hydrolysis reaction should be conducted at not higher than pH 7. While, too low pH tends to cause the deactivation of enzymes. Thus, it is preferred that the reaction is conducted in the range of pH 3.5 to pH 6.3. Further, to keep the pH at the level, utilization of a buffer solution is desirable to prevent the lowering of pH value due to the formation of an organic acid, such as acetic acid, during the course of hydrolysis. As the buffer solution, either inorganic or organic acid salt buffer may be employed.

The concentration of the ester employed as the substrate may be in the range of 1 to 80% (w/w), preferably 5 to 35% (w/w), of the reaction mixture.

Then, after the asymmetric hydrolysis reaction, the free (S)-(−)-α-cyano-3-phenoxybenzyl alcohol and the unreacted ester of the antipode are separated from the reaction mixture. For that purpose, solvent extraction, liquid phase separation by standing or other procedures, can be adequately employed. For example, the reaction mixture may be extracted with an organic solvent, such as benzene, toluene, etc., and the extract is subjected to fractionating distillation in vacuo to separate (S)-(−)-α-cyano-3-phenoxy-benzyl alcohol from the ester of its antipode. Alternatively, the extract may be subjected to a column chromatography using, for example, silica gel etc. to separate free (S)-(−)-α-cyano-3-phenoxybenzyl alcohol.

On the other hand, the unreacted ester thus separated may be used again as the starting material of the method of the present invention after the epimerization by contact with a basic compound, such as ammonia, pyridine, triethylamine, etc.

Hereunder, the present invention will be more fully described by means of the following examples, which, however, should not be construed to be limitative.

Examples 1 to 4

To 10 ml of 0.2 M concentration of an acetate buffer solution (pH 5.0) were added 1.0 g of (R,S)-α-cyano-3-phenoxybenzyl acetate and each 20 mg of an esterase illustrated in Table 1. The mixture was vigorously agitated by means of a magnetic stirrer at 30°C to advance the reaction. After 24 hours, the reaction mixture was extracted with toluene. The extract was analyzed by high-performance liquid chromatography (HPLC) (Lichrosorb RP-18, MeOH-water (6:4), 254 nm, UV detection), and the conversion was calculated from the peak area ratio of unreacted α-cyano-3-phenoxybenzyl acetate and free α-cyano-3-phenoxybenzyl alcohol. The HPLC analysis also recognized that no 3-phenoxybenzaldehyde had been by-produced during the course of the reaction under the above conditions. The toluene extract was concentrated, and subjected to a silica gal chromatography and eluted with cyclohexane-diethyl ether (94:6) mixture to isolate the unreacted acetate of α-cyano-3-phenoxybenzyl alcohol. The column was then eluted with methanol containing a trace $(10^{-5}\%)$ of p-toluenesulfonic acid, to obtain free α-cyano-3-phenoxybenzyl alcohol. Its structure was confirmed by HPLC, NMR and IR observations.

Next, the solvent was evaporated away from the elute. Taking a part of the residual alcohol, its specific rotation was measured in chloroform. As the results, it was confirmed that the obtained α-cyano-3-phenoxybenzyl alcohol had been (−)-isomer, i.e. (S)-(−)-α-cyano-3-phenoxybenzyl alcohol. (see Table 1).

While, 10 mg of the obtained free α-cyano-3-phenoxybenzyl alcohol was dissolved in 1 ml of toluene, and reacted with the equal mole amount of (S)-(+)-2-(4-chlorophenyl)-isovaleric acid chloride in the presence of pyridine to convert it to a diastereomer of α-cyano-3-phenoxybenzyl (S)-(+)-2-(4-chloro-phenyl)-isovalerate, which was analyzed for the optical isomer ratio by gas chromatography (10% DCQF-1, 2.5 m, 250°C), with the results as shown in the following Table 1.

On the other side, the specific rotation of the unreacted ester recovered from the column after the enzymatic reaction was found to exhibit the negative value in chloroform (for example, $[\alpha]_D = -8.5$ (c=1.5) in Example 1)

TABLE 1

| Example No. | Origin of esterase (name of enzyme) | Conversion (%) | Free α-cyano-3-phenoxybenzyl alcohol | |
|---|---|---|---|---|
| | | | Spec. rotation $[\alpha]_D^{25}$, in $CHCl_3$ | Optical isomer ratio(%) [(S)-(−)-isomer: (R)-(+)-isomer] |
| 1 | *Arthrobacter* sp. (lipase Godo BSL) | 49.8 | −24.5° (c=1.2) | >99:<1 |
| 2 | *Pseudomonas* sp. (lipase "Amano") | 34.0 | −22.2° (c=1.4) | 93:7 |
| 3 | *Aspergillus* sp. (lipase AP) | 47.9 | −16.2° (c=1.4) | 79:21 |
| 4 | *Alcaligenes* sp. (lipase PL No. 266) | 48.9 | −17.2° (c=0.7) | 82:18 |

Examples 5 to 10

The same procedures as in Examples 1 to 4 were repeated with the exceptions that the amount of each esterase was 40 mg instead of 20 mg, that the buffer solution was 0.1 M concentration of a phosphate buffer solution (pH 6.0) instead of the 0.2 M acetate buffer solution (pH 5.0), and that the pH was controlled at the constant level using a pH controller by careful addition of an aqueous 1 M NaOH solution in the form of finely divided water drops using a drop controller. After the enzymatic reaction followed by separation of the reaction product, the resulting (S)-(−)-α-cyano-3-phenoxybenzyl alcohol was measured for specific rotation and conversion, with the results as shown in Table 2.

TABLE 2

| Example No. | Origin of esterase (name of enzyme) | Conversion (%) | Free α-cyano-3-phenoxybenzyl alcohol ($[\alpha]_D^{25}$, in $CHCl_3$) |
|---|---|---|---|
| 5 | *Achromobacter* sp. (lipase AL) | 43.7 | −16.0° (c=1.0) |
| 6 | *Alcaligenes* sp. (lipase PL No. 679) | 52.0 | −17.5° (c=1.2) |
| 7 | *Pseudomonas* sp. (lipase "Amano") | 49.7 | −22.5° (c=1.3) |
| 8 | *Mucor* sp. (lipase M-AP) | 19.2 | −14.2° (c=0.8) |
| 9 | Hog pancreas (steapsin) | 33.0 | −15.2° (c=0.8) |
| 10 | Hog pancreas (pancreatin) | 30.0 | −15.4° (c=0.8) |

Examples 11 to 19

To 10 ml of 0.2 M concentration of an acetate buffer solution (pH 5.4) were added 1.5 g of (R,S)-α-cyano-3-phenoxybenzyl caprate and each 30 mg of an esterase described in Table 3. The mixture was vigorously agitated for 24 hours at 30°C. Thereafter, the separation and analyses were conducted according to the same procedures as in Examples 1 to 4, with the results shown in Table 3.

6

TABLE 3

| Example No. | Origin of esterase (name of enzyme) | Conversion (%) | Free α-cyano-3-phenoxybenzyl alcohol, sp. rotat. ($[\alpha]_D^{25}$, in $CHCl_3$) |
|---|---|---|---|
| 11 | *Arthrobacter* sp. (lipase Godo BSL) | 50.1 | −23.0° (c=1.2) |
| 12 | *Alcaligenes* sp. (lipase PL No. 266) | 49.7 | −19.1° (c=1.0) |
| 13 | *Alcaligenes* sp. (lipase PL No. 679) | 33.4 | −18.5° (c=1.0) |
| 14 | *Achromobacter* sp. (lipase AL) | 27.7 | −17.5° (c=1.0) |
| 15 | *Aspergillus* sp. (lipase AP) | 47.8 | −16.3° (c=1.2) |
| 16 | *Pseudomonas* sp. (lipase "Amano") | 39.5 | −22.2° (c=1.2) |
| 17 | *Mucor* sp. (lipase M-AP) | 11.2 | −14.1° (c=1.0) |
| 18 | Hog pancreas (steapsin) | 22.3 | −15.5° (c=0.8) |
| 19 | Hog pancreas (pancreatin) | 17.6 | −14.9° (c=0.8) |

Examples 20 to 25

The same procedures as in Examples 1 to 4 were repeated with the exceptions that the amount of each esterase was 30 mg instead of 20 mg, that the buffer solution was 0.1 M concentration of an acetate buffer solution (pH 5.3) instead of the 0.2 M acetate buffer solution, that the pH was controlled at the constant level using a pH controller by careful addition of an aqueous 1 M NaOH solution in the form of finely divided water drops using a drop controller, and that the amount of the substrate, acetate of (R,S)-α-cyano-3-phenoxybenzyl alcohol, employed was varied from 1.0 g to the values set forth in Table 4. After the enzymatic reaction, followed by separation of the reaction product, the product was measured for conversion, specific rotation and optical isomer ratio by gas chromatography, with the results as shown in Table 4.

TABLE 4

| Example No. | Origin of esterase (name of enzyme) | Amount of substrate (g) | Conversion (%) | Free α-cyano-3-phenoxybenzyl alcohol | |
|---|---|---|---|---|---|
| | | | | Specific rotation ($[\alpha]_D^{25}$, in CHCl$_3$) | Optical isomer ratio [(S)-(−)-isomer: (R)-(+)-isomer] |
| 20 | *Arthrobacter* sp. (lipase Godo BSL) | 2.0 | 50.0 | −24.8° (c=1.2) | 100:0 |
| 21 | *Arthrobacter* sp. (lipase Godo BSL) | 3.0 | 49.7 | −23.8° (c=1.2) | 99:1 |
| 22 | *Arthrobacter* sp. (lipase Godo BSL) | 5.0 | 43.6 | −22.0° (c=1.4) | 93.5:6.5 |
| 23 | *Pseudomonas* sp. (lipase "Amano") | 2.5 | 41.1 | −23.2° (c=1.2) | 99:1 |
| 24 | *Alcaligenes* sp. (lipase PL No. 266) | 4.0 | 52.4 | −17.5° (c=1.3) | 83:17 |
| 25 | *Aspergillus* sp. (lipase AP) | 2.0 | 47.4 | −16.4° (c=1.3) | 78:22 |

Examples 26 to 31

In a 1000 ml Erlenmeyer flask was put 200 ml of a bouillon medium (prepared by dissolving 5.0 g peptone, 5.0 g meat extract and 3.0 g NaCl in 1 l of water, adding 2 g of tributyrin, in cases of Examples 26 to 28, or 2 g of olive oil, in cases of Examples 29 to 31, and adjusting the pH to 7.2 with Na$_2$HPO$_4$ and KH$_2$PO$_4$). After sterilization, the medium was inoculated with 2 platinum loops of a slant cultured microorganism as illustrated in Table 5, and cultured on a reciprocating shaker at 30°C for 48 hours. The cultured medium was then centrifuged, and acetone was added to the aqueous solution layer to make 85% concentration. The mixture was immediately filtered, and the separated precipitate was washed with water and dissolved into 15 ml of 0.2 M concentration of an acetate buffer solution (pH 5.2). To the solution was added 1.5 g of butyrate, in Examples 26 to 28 or 3.0 g of laurate, in Examples 29 to 31, of (R,S)-α-cyano-3-phenoxybenzyl alcohol. The mixture was vigorously agitated at 33°C for 26 hours to make the reaction proceed. Thereafter, the separation and analyses were conducted as in Examples 1 to 4, and the conversion and specific rotation were measured with the results as shown in Table 5.

TABLE 5

| Example No. | Origin of esterase (name of micro-organism cultured) | Conversion (%) | Free α-cyano-3-phenoxybenzyl alcohol, sp. rotat. ($[\alpha]_D^{25}$, in CHCl$_3$) |
|---|---|---|---|
| 26 | *Arthrobacter simplex* IFO 3530 | 38.7 | −23.0° (c=0.9) |
| 27 | *Pseudomonas fragi* IFO 3458 | 24.3 | −21.9° (c=0.9) |
| 28 | *Alcaligenes faecalis* IFO 12669 | 32.3 | −14.2° (c=0.9) |
| 29 | *Arthrobacter simplex* IFO 3530 | 49.1 | −23.5° (c=1.0) |
| 30 | *Pseudomonas fragi* IFO 3458 | 39.1 | −22.0° (c=1.0) |
| 31 | *Pseudomonas fluorescens* IFO 3081 | 32.2 | −18.0° (c=1.0) |

Example 32

To 2 ml of 0.1 M concentration of an acetate buffer solution (pH 4.5) were added 8.0 g of (R,S)-α-cyano-3-phenoxybenzyl acetate and 160 mg of esterase from *Arthrobacter* sp. (lipase Godo BSL). The mixture was vigorously agitated using an agitation piece at 40°C to make the reaction proceed. During the reaction, the pH was controlled at the constant level using a pH controller by careful addition of an aqueous 1 M NaOH solution in the form of finely divided water drops, using a drop controller. After 24 hours reaction, the reaction product was extracted with toluene.

Thereafter, the same procedures as in Examples 1 to 4 were followed, with the results shown in Table 6.

TABLE 6

| | | Free α-cyano-3-phenoxybenzyl alcohol | |
| | | | |
| Example No. | Conversion (%) | Specific rotation ($[α]_D^{25}$, in $CHCl_3$) | Optical isomer ratio (%) [(S)-(−)-isomer: (R)(+)-isomer] |
|---|---|---|---|
| 32 | 50.0 | −24.5° (c=1.2) | >99:<1 |

Example 33

To 2 ml of 0.2 M concentration of an acetate buffer solution (pH 4.5) were added 8.0 g of (R,S)-α-cyano-3-phenoxybenzyl acetate and 20 mg of esterase from *Chromobacterium* sp. (lipase Toyo). The mixture was stirred at 30°C to make the reaction proceed. During the reaction, the pH was controlled at the constant level using a pH controller by careful addition of an aqueous 1 M NaOH solution in the form of finely divided water drops using a drop controller. After 24 hour reaction, the reaction product was extracted with toluene. Thereafter, the same procedures as in Examples 1 to 4 were followed, with the results shown in Table 7.

TABLE 7

| | | Free α-cyano-3-phenoxybenzyl alcohol | |
| | | | |
| Example No. | Conversion (%) | Specific rotation ($[α]_D^{25}$, in $CHCl_3$) | Optical isomer ratio (%) [(S)-(−)-isomer: (R)-(+)-isomer] |
|---|---|---|---|
| 33 | 47.7 | −23.5° (c=1.0) | 98.0:2.0 |

Examples 34 to 63

In a 500 ml flask having shoulder was put 100 ml of liquid medium [a sugared bouillon medium (prepared by dissolving 10.0 g of glucose, 5.0 g of peptone, 5.0 g of meat extract and 3.0 g of NaCl in 1 l of water and adjusting to pH 7.2) for bacteria as in Examples 34 to 37 and 42 to 49, or a malt extract-yeast extract medium (prepared by dissolving 5.0 g of peptone, 10.0 g of glucose, 3.0 g of malt extract and 3.0 g of yeast extract in 1 l of water, and adjusting to pH 7.2) for fungi and yeasts as in Examples 38 to 41 and 50 to 63. After sterilization, the medium was inoculated with 2 platinum loops of a slant cultured microorganism as illustrated in Table 8, and cultured on a reciprocating shaker at 30°C for 72 hours. The pH value of the cultured medium was adjusted to pH 5.0 by use of an aqueous 2 M HCl solution. To the cultured medium was added 2.0 g of (R,S)-α-cyano-3-phenoxybenzyl acetate, and the mixture was agitated at 30°C for 30 hours to make the reaction proceed. During the reaction, the pH was controlled at the constant level in the similar way to Example 33.

Thereafter, the reaction product was separated as in Examples 1 to 4, and the obtained α-cyano-3-phenoxybenzyl alcohol was measured for the optical isomer ratio and conversion, with the results shown in Table 8.

9

## 0 080 827

TABLE 8

| Example No. | Origin of esterase (microorganism cultured) | Conversion (%) | Free α-cyano-3-phenoxybenzyl alcohol, optical isomer ratio (S)-(−)-isomer:(R)-(+)-isomer |
|---|---|---|---|
| 34 | *Bacillus cereus* (ATCC-13366) | 50.3 | 87.3:12.7 |
| 35 | *Bacillus licheniformis* (IFO-12197) | 18.5 | 96.0: 4.0 |
| 36 | *Micrococcus luteus* (IFO-3066) | 25.9 | 79.5:20.5 |
| 37 | *Nocardia erythropolis* (IFO-12320) | 39.2 | 88.4:11.6 |
| 38 | *Rhodoturula minuta* (IFO-0387) | 47.1 | 89.1:10.9 |
| 39 | *Rhodoturula minuta* var. *texensis* (IFO-0879) | 49.6 | 96.5: 3.5 |
| 40 | *Torulopsis candida* (IFO-0380) | 17.0 | 88.3:11.7 |
| 41 | *Candida utilis* (IFO-1086) | 19.0 | 75.4:24.6 |
| 42 | *Chromobacterium violaceum* (IFO-12614) | 27.4 | 90.5: 9.5 |
| 43 | *Brevibacterium ammoniagenes* (IFO-12072) | 43.9 | 89.2:10.8 |
| 44 | *Enterobacter cloacae* (IFO-3320) | 23.1 | 85.7:14.3 |
| 45 | *Flavobacterium arborescens* (IFO-3750) | 26.5 | 64.6:45.4 |
| 46 | *Mycobacterium phlei* (IFO-3158) | 31.7 | 77.3:22.7 |
| 47 | *Corynebacterium equi* (ATCC-7699) | 34.3 | 72.3:27.6 |
| 48 | *Lactobacillus casei* (IFO-3322) | 13.9 | 76.1:23.9 |
| 49 | *Streptomyces griseus* (IFO-3356) | 25.7 | 77.5:22.5 |
| 50 | *Trichoderma viride* (IFO-4847) | 48.0 | 81.9:18.1 |
| 51 | *Penicillium freguentans* (IFO-5692) | 30.0 | 82.7:17.3 |
| 52 | *Rhizopus chinensis* (IFO-4737) | 39.7 | 89.0:11.0 |

TABLE 8 (continued)

| Example No. | Origin of esterase (microorganism cultured) | Conversion (%) | Free α-cyano-3-phenoxybenzyl alcohol, optical isomer ratio (S)-(−)-isomer:(R)-(+)-isomer |
|---|---|---|---|
| 53 | *Aureobasidium pullulans* (IFO-4464) | 26.2 | 80.1:19.9 |
| 54 | *Actinomucor elegans* (IFO-4022) | 37.5 | 88.2:11.8 |
| 55 | *Gibberella zeae* (IFO-7160) | 11.8 | 85.5:14.5 |
| 56 | *Geotrichum candidum* (IFO-5368) | 35.8 | 83.0:17.0 |
| 57 | *Absidia hyalospora* (IFO-8082) | 10.7 | 77.4:22.6 |
| 58 | *Cunninghamella elegans* (IFO-6334) | 25.9 | 79.7:20.3 |
| 59 | *Gliocladium roseum* (IFO-5422) | 8.9 | 83.1:16.9 |
| 60 | *Saccharomyces rouxii* (IFO-0505) | 17.5 | 67.8:32.2 |
| 61 | *Cryptococcus albidus* (IFO-0378) | 13.6 | 69.1:30.9 |
| 62 | *Pichia polymorpha* (IFO-1166) | 32.8 | 75.7:24.3 |
| 63 | *Hansenula anomala* (IFO-0117) | 40.1 | 77.4:22.6 |

Example 64

One liter of a cultured medium of *Rhodotorula minuta* var. *texensis* (IFO-0879) prepared in the same way as in Example 39 was centrifuged. The collected cells were washed twice with distilled water and then freeze-dried. To 10 ml of 0.2 M concentration of an acetate buffer solution (pH 5.0) were dissolved 500 mg of the freeze-dried cells and 3.0 g of (R,S)-α-cyano-3-phenoxybenzyl butyrate. The mixture was agitated at 40°C for 20 hours to advance the reaction. Thereafter, the separation and analysis were conducted according to the procedures similar to those in Examples 1 to 4. The resulting free α-cyano-3-phenoxy-benzyl alcohol was measured for conversion and optical isomer ratio, with the results shown in Table 9.

TABLE 9

| Example No. | Conversion (%) | Free α-cyano-3-phenoxybenzyl alcohol, optical isomer ratio [(S)-(−)-isomer:(R)-(+)-isomer] |
|---|---|---|
| 64 | 50.0 | 98.3:1.7 |

Example 65

Into 2 l jar fermenter was placed 1 l of an olive oil-added bouillon medium (prepared by dissolving 5.0 g of peptone, 5.0 g of meat extract and 3.0 g of NaCl in 1 l of water, adding 2.0 g of olive oil thereto, and adjusting to pH 7.0. After sterilization, the medium was inoculated with *Bacillus cereus* (ATCC-13366), and cultured under aeration-agitation at 30°C for 72 hours. Then, the medium was centrifuged, and the aqueous layer was concentrated to 3 times concentration. Acetone was added to the concentrate to make the 85% concentration. The mixture was immediately filtered, and the obtained precipitate was washed with water and dissolved into 10 ml of 0.2 M concentration of an acetate buffer solution (pH 4.5). To the solution was added 5.0 g of (R,S)-α-cyano-3-phenoxybenzyl laurylate, and the mixture was vigorously agitated for 16 hours at 40°C to advance the reaction. Thereafter, the separation and analyses were conducted according to the procedures similar to those in Examples 1 to 4. Conversion and optical isomer ratio of free α-cyano-3-phenoxybenzyl alcohol were measured, with the results shown in Table 10.

TABLE 10

| Example No. | Conversion (%) | Free α-cyano-3-phenoxybenzyl alcohol, optical isomer ratio [(S)-(−)-isomer:(R)-(+)-isomer] |
|---|---|---|
| 65 | 46.6 | 96.9:3.1 |

## Claims

1. A method for biotechnologically preparing (S)-(−)-α-cyano-3-phenoxybenzyl alcohol, characterized in that an organic carboxylic acid (saturated or unsaturated, having 1 to 18 carbon atoms) ester of (R,S)-α-cyano-3-phenoxybenzyl alcohol is reacted with an esterase originated from a microorganism or an animal pancreas or liver, which enzyme is capable of asymmetrically hydrolyzing the ester of the (S)-(−)-α-cyano-3-phenoxybenzyl alcohol predominantly, at a pH not higher than 7 to asymmetrically hydrolyze the ester to give the (S)-(−)-α-cyano-3-phenoxybenzyl alcohol and the ester of its antipode.

2. A method according to claim 1, wherein the esterase is originated from a microorganism belonging to genus *Arthrobacter, Alcaligenes, Achromobacter, Pseudomonas, Aspergillus, Mucor, Chromobacterium, Bacillus, Micrococcus, Candida, Torulopsis, Nocardia, Rhodotorula, Brevibacterium, Enterobacter, Flavobacterium, Mycobacterium, Corynebacterium, Lactobacillus, Streptomyces, Trichoderma, Penicillium, Rhizopus, Aureobasidium, Actinomucor, Gibberella, Geotricum, Absidia, Cunninghamella, Gliocladium, Saccharomyces, Cryptococcus, Pichia* or *Hansenula*.

3. A method according to claim 1 or 2, wherein the asymmetric hydrolysis reaction is conducted by stirring or shaking vigorously a mixture of the ester and an esterase-containing liquor.

4. A method according to claim 1, 2 or 3, wherein the ester is the ester of an organic carboxylic acid having 2 to 12 carbon atoms.

5. A method according to any one of the preceding claims, wherein the asymmetric hydrolysis reaction is conducted at pH 3.5 to pH 6.3.

6. A method according to any one of the preceding claims, wherein the reaction temperature is from 20°C to 50°C.

7. A method according to any one of the preceding claims, wherein the concentration of the ester is from 5 to 35% (w/w) of the reaction mixture.

8. A method according to any one of the preceding claims wherein the esterase is contained in microorganism cells or in a culture medium or filtrate thereof.

9. A method according to anyone of the preceding claims comprising the additional step of converting the resulting (S)-(−)-α-cyano-3-phenoxybenzyl alcohol into a synthetic pyrethroid in manner known *per se*.

## Patentansprüche

1. Eine Methode zur biotechnologischen Herstellung des (S)-(−)-α-Cyan-3-phenoxybenzylalkohols, dadurch gekennzeichnet, daß der Ester von (R,S)-α-Cyan-3-phenoxybenzylalkohol und einer organischen Carbonsäure (gesättigt oder ungesättigt, mit 1 bis 18 Kohlenstoffatomen) mit einer Esterase, die von einem Mikroorganismus oder aus einem tierischen Pankreas oder aus tierischer Leber stammt, welches Enzym in der Lage ist, den Ester von (S)-(−)-α-Cyan-3-phenoxybenzylalkohol zur Hauptsache asymmetrisch zu hydrolysieren, bei einem pH-Wert von nicht mehr als 7 umsetzt, um so den Ester unter Bildung des (S)-(−)-α-Cyan-3-phenoxybenzylalkohols und des Esters seines Antipoden asymmetrisch zu hydrolysieren.

2. Eine Methode gemäß Anspruch 1, in welcher die Esterase von einem Mikroorganismus stammt, welcher zu einer der nachstehend genannten Genii gehört: Arthrobacter, Alcaligenes, Achromobacter, Pseudomonas, Aspergillus, Mucor, Chromobacterium, Bacillus, Micrococcus, Candida, Torulopsis, Nocardia, Rhodotorula, Brevibacterium, Enterrobacter, Flavobacterium, Mycobacterium, Corynebacterium, Lactobacillus, Streptomyces, Trichoderma, Penicillium, Rhizopus, Aureobasidium, Actinomucor, Gibberella, Geotricum, Absidia, Cunninghamella, Gliocladium, Saccharomyces, Cryptococcus, Pichia oder Hansenula.

3. Eine Methode gemäß Anspruch 1 oder 2, in welcher die asymmetrische hydrolytische Reaktion durchgeführt wird, indem man eine Mischung aus dem Ester und einem esterasehaltigen flüssigen Medium kräftig rührt oder schüttelt.

4. Eine Methode gemäß Anspruch 1, 2 oder 3, in welcher der Ester der Ester einer organischen Carbonsäure mit 2 bis 12 Kohlenstoffatomen ist.

5. Eine Methode gemäß irgendeinem der vorhergehenden Ansprüche, in welcher die asymmetrische hydrolytische Reaktion bei einem pH-Wert von 3.5 bis 6.3 durchgeführt wird.

6. Eine Methode gemäß irgendeinem der vorhergehenden Ansprüche, gemäß welcher die Temperatur im Bereich von 20°C bis 50°C liegt.

7. Eine Methode gemäß irgendeinem der vorhergehenden Ansprüche, in welcher die Konzentration des Esters 5 bis 35 Gewichtsprozent, bezogen auf das Gewicht der Reaktionsmischung, beträgt.

8. Eine Methode gemäß irgendeinem der vorhergehenden Ansprüche, in welcher die Esterase in Mikroorganismuszellen oder in einem Züchtungsmedium oder einem Filtrat davon enthalten ist.

9. Eine Methode gemäß irgendeinem der vorhergehenden Ansprüche, umfassend den zusätzlichen Schritt des Umwandelns des erhaltenen (S)-(−)-α-Cyan-3-phenoxybenzylalkohols in an sich bekannter Weise in ein synthetisches Pyrethroid.

**Revendications**

1. Procédé de préparation biotechnologique de l'alcool (S)-(−)-α-cyano-3-phénoxybenzylique, caractérisé en ce qu'il consiste à faire réagir un ester (R,S)-α-cyano-3-phénoxybenzylique d'un acide carboxylique organique (saturé ou insaturé, ayant de 1 à 18 atomes de carbone) sur une estérase provenant d'un microorganisme ou d'un pancréas ou d'un foie d'animal, cette enzyme étant susceptible d'hydrolyser asymétriquement l'ester de l'alcool (S)-(−)-α-cyano-3-phénoxybenzylique d'une manière prépondérante, à un pH qui n'est pas supérieur à 7, pour hydrolyser asymétriquement l'ester en l'alcool (S)-(−)-α-cyano-3-phénoxybenzylique et en l'ester de son antipode.

2. Procédé suivant la revendication 1, dans laquelle l'estérase provient d'un microorganisme appartenant aux genres *Arthrobacter, Alcaligenes, Achromobacter, Pseudomonas, Aspergillus, Mucor, Chromobacterium, Bacillus, Micrococcus, Candida, Torulopsis, Nocardia, Rhodotorula, Brevibacterium, Enterobacter, Flavobacterium, Mycobacterium, Corynebacterium, Lactobacillus, Strepto-Streptomyces, Trichoderma, Penicillium, Rhizopus, Aureobasidium, Actinomucor, Gibberella, Geotricum, Absidia, Cunninghamella, Gliocladium, Saccharomyces, Cryptococcus, Pichia* ou *Hansenula*.

3. Procédé suivant la revendication 1 ou 2, qui consiste à effectuer la réaction d'hydrolyse asymétrique en brassant ou en agitant vigoureusement un mélange de l'ester et d'une liqueur contenant une estérase.

4. Procédé suivant la revendication 1, 2 ou 3, dans lequel l'ester est l'ester d'un acide carboxylique organique ayant de 2 à 12 atomes de carbone.

5. Procédé suivant l'une quelconque des revendications précédentes, qui consiste à effectuer la réaction d'hydrolyse asymétrique à un pH de 3,5 à 6,3.

6. Procédé suivant l'une quelconque des revendications précédentes, dans lequel la température réactionelle est comprise entre 20°C et 50°C.

7. Procédé suivant l'une quelconque des revendications précédentes, dans lequel la concentration de l'ester est comprise entre 5 et 35% (poids/poids) du mélange réactionnel.

8. Procédé suivant l'une quelconque des revendications précédentes, dans lequel l'estérase est contenue dans des cellules de microorganismes ou dans un milieu de culture ou dans un filtrat de ceux-ci.

9. Procédé suivant l'une quelconque des revendications précédentes, caractérisé en ce qu'il comprend le stade supplémentaire de transformation de l'alcool (S)-(−)-α-cyano-3-phénoxybenzylique obtenu, en un pyréthroïde synthétique, d'une manière en soi connue.